Europäisches Patentamt

European Patent Office

Office européen des brevets

(11) Veröffentlichungsnummer: **0 301 250**
**A1**

(12) **EUROPÄISCHE PATENTANMELDUNG**

(21) Anmeldenummer: 88110246.1

(51) Int. Cl.⁴: **A01N 1/02 , A61K 35/18**

(22) Anmeldetag: 28.06.88

(30) Priorität: **11.07.87 DE 3722984**

(43) Veröffentlichungstag der Anmeldung:
**01.02.89 Patentblatt 89/05**

(84) Benannte Vertragsstaaten:
**AT BE CH DE ES FR GB GR IT LI LU NL SE**

(71) Anmelder: **BIOTEST PHARMA GMBH**
**Landsteiner Strasse 5**
**D-6072 Dreieich(DE)**

(72) Erfinder: **Gänshirt, Karlheinz, Dr.Dipl.-Chem.**
**August-Bebel-Strasse 34**
**D-6072 Dreieich(DE)**
Erfinder: **Handel, Klaus Dieter, Dr.Dipl.-Ing.**
**Pallaswiesenstrasse 14**
**D-6100 Darmstadt(DE)**
Erfinder: **Walker, Wolfram H., Dr.Dipl.-Chem.**
**Thomastrasse 8**
**D-6074 Rödermark(DE)**
Erfinder: **Von Eisenhart-Rothe, Bolko, Dr.**
**Hünenkamp 2 h**
**D-2000 Schenefeld(DE)**

(74) Vertreter: **Wolff, Hans Joachim, Dr.jur.**
**Dipl.-Chem. et al**
**Beil, Wolff & Beil Rechtsanwälte Postfach 80**
**01 40 Adelonstrasse 58**
**D-6230 Frankfurt am Main 80(DE)**

(54) **Wässrige Lösung zum Suspendieren und Lagern von Zellen, insbesondere Erythrozyten.**

(57) Zur Verbesserung der Sauerstoffabgabe der Erythrozyten in einem Erythrozytenkonzentrat bei Beibehaltung einer guten und langen Lagerungsfähigkeit und geringen Hämolyserate verwendet man eine wässrige Lösung zum Suspendieren und Lagern von Zellen, insbesondere Erythrozyten, die Nährstoffe, z.B. Zucker u.a. und nicht in die Zellen eindringende, in wässriger Lösung lösliche Substanzen, z.B. Zuckeralkohole u.a. enthält, wobei die nicht in die Zellen eindringende Substanz in einer Konzentration von mehr als 110 mmol/l vorliegt.

EP 0 301 250 A1

## Wässrige Lösung zum Suspendieren und Lagern von Zellen, insbesondere Erythrozyten

Die Erfindung betrifft eine wässrige Lösung zum Suspendieren und Lagern von Zellen, insbesondere Erythrozyten, enthaltend mindestens einen Nährstoff und mindestens eine nicht in die Zellen eindringende, in wässriger Lösung lösliche Substanz.

Im Rahmen der Blutkomponententherapie wird aus stabilisiertem Vollblut (Blutkonserve) durch Abtrennen des Plasmas ein Erythrozytenkonzentrat hergestellt. Dieses Erythrozytenkonzentrat wird heute in einer grossen Zahl von therapeutischen Anwendungen eingesetzt, bei denen die Indikation Anämie bzw. Erythrozytenzufuhr besteht. Da durch die Abtrennung des Plasmas auch ein Teil der einer Blutkonserve üblicherweise zugesetzten Blutstabilisatorlösung entfernt wird, ist man dazu übergegangen, die Konzentrationen bestimmter Bestandteile dieser Stabilisatorlösung zu erhöhen, um zumindest dieselbe Lagerfähigkeit des Erythrozytenkonzentrates wie die für die Blutkonserve zu erhalten. Es ist ausserdem bekannt, dass durch eine nachträgliche Zugabe einer Lösung zum Suspendieren zu dem Erythrozytenkonzentrat die ursprünglich hergestellten Konzentrate durch diese Suspensionslösung verdünnt werden, so dass eine erwünschte niedrige Viskosität erreicht und dadurch die Transfusion dieser Lösung erleichtert wird. Sie ermöglicht ausserdem, spezifische für die Lagerungsfähigkeit der Erythrozyten notwendige Substanzen zuzuführen, um deren Überlebensrate in vitro und in vivo zu verlängern.

Als Lösung zum Suspendieren und Lagern von Zellen, insbesondere Erythrozyten, eignen sich verschiedene Lösungen. So wird z.B. in der US-PS 4 267 269 eine Lösung beschrieben, die neben Natriumchlorid, Glucose oder Fructose und Adenin, den Zuckeralkohol Mannit in Mengen von 250 bis 2 000 mg/100 ml = ca. 13,7 bis 109,9 mmol/l, vorzugsweise 500 bis 1 000 mg/100 ml = ca. 27 bis 55 mmol/l enthält.

In der DE-OS 32 408 wird eine Lösung beschrieben, die ebenfalls Natriumchlorid, Glucose oder Fructose und Adenin, jedoch als Zuckeralkohol Sorbit bzw. Xylit in Mengen von 1 bis 20 g/l = ca. 27 bis 109,9 mmol/l, vorzugsweise 10 g/l = ca. 55 mmol/l und ggf. zusätlich Guanosin enthält. Diese Lösung verbessert die Überlebensrate und reduziert die während der Lagerung auftretende Hämolyse der Erythrozyten.

Zwar wurde durch die Zugabe solcher Suspensionslösungen zu Erythrozytenkonzentraten die Lagerfähigkeit und damit die Überlebensrate der Erythrozyten verbessert, jedoch waren diese Verbesserung und insbesondere die Sauerstoffabgabe der Erythrozyten nicht voll zufriedenstellend.

Der Erfindung lag die Aufgabe zugrunde, bei Beibehaltung der guten Lagerungsfähigkeit und geringen Hämolyserate die Sauerstoffabgabe der Erythrozyten in einem Erythrozytenkonzentrat zu verbessern. Diese Aufgabe wird erfindungsgemäss dadurch gelöst, dass man eine gattungsgemässe Lösung verwendet, in der die nicht in die Zellen eindringende in wässriger Lösung lösliche Substanz in einer Konzentration von mehr als 110 mmol/l vorliegt.

Gegenüber bisher bekannten Lagerlösungen, die wie vorstehend beschrieben, etwa 5 bis 110 mmol Mannit bzw. Sorbit oder Xylit als nicht in die Zellen eindringende wasserlösliche Substanzen enthielten, stellt die erfindungsgemäss verwendete Menge dieser Substanzen eine beachtliche Konzentrationserhöhung dar. Bisher wurden in Lagerlösungen Konzentrationen von max. 110 mmol/l für ausreichend gehalten. Diese Konzentration wurde gewählt, um die Hämolyse der Erythrozyten zu erniedrigen. Eine weitere Erhöhung brachte keine weitere Verbesserung bezüglich der Hämolyse.

Überraschenderweise wurde gefunden, dass bei Verwendung von Konzentrationen dieser Substanz, die über den bisher üblichen liegen, eine Verbesserung des 2,3-Di-phosphoglycerat(2,3-DPG)-Gehaltes auftritt. Der Abfall war gegenüber bisherigen Lösungen um ca. 10-15 Tage später.

Der 2,3-DPG-Gehalt ist ein Maß für die Sauerstoffabgabe der Erythrozyten. Je höher er ist, desto besser ist die Abgabe des Sauerstoffes in das durchströmte Gewebe.

Zu den "nicht in die Zellen eindringenden in wässriger Lösung löslichen Substanzen" gehören z.B. Zuckeralkohole, wie Sorbit, Mannit oder Xylit; Citrate; Disaccharide sowie Plasmaproteine. Ohne sich auf eine bestimmte Theorie festzulegen, glaubt man, dass Stoffe, die die Eigenschaft von Plasmaproteinen, nämlich nicht in die Zellen einzudringen, aufweisen und wasserlöslich sind, erfindungsgemäss zum Einsatz kommen können (vgl. auch Högman et. al Vox Sang. 41: 274-281, 280 r. Sp. (4) (1981)).

Der wesentliche Faktor bei der Konzentration der nicht in die Zellen eindringenden Substanz ist die Teilchenzahl und der daraus resultierende osmotische Druck und nicht das Gewicht der Substanz. Bei Mannit und Sorbit mit einem Molekulargewicht von 182 entsprechen 20 g/l etwa 109,9 mmol bzw. mosmol. Bei einer Substanz mit einem höheren Molekulargewicht werden somit grössere Gewichtsmengen einzusetzen sein und umgekehrt. Nach oben hin sind den Mengen der Susbtanz keine Grenzen gesetzt. Falls man isotone Lösungen wünscht, sollte die Grenze zur Erreichung der Isotonie nicht überschritten werden. Diese liegt bei Sorbit bei etwa 310 mmol. Bei hypertonen Lösungen können wesentlich größere Mengen verwendet werden. Lediglich der Eintritt einer zellschädigenden Wirkung und/oder die Löslichkeitsgrenze der jeweiligen Susbstanz setzen eine Grenze nach oben. Dieser Wert wird jedoch vom Fachmann in jedem Falle mit einfachen Mitteln zu ermitteln sein.

Zu den einsetzbaren Nährstoffen gehören alle Zucker, wie z.B. Glucose, Fructose, Mannose und Galactose sowie auch Inosin (vgl. Ernest Beutler, Red cell metabolism, A manual of Biochemical Methods, Grune & Stratton, S. 3-6, insbes. S. 6 (1975)).

Die durch die hohe Konzentration der nicht in die Zellen eindringenden Substanz erzielte vorteilhafte Eigenschaft der Lösung lässt sich durch Zugabe von Adenin und Guanosin noch weiter verbessern. Durch die Zugabe von Hydrogenphosphat und Dihydrogenphosphat wird die Lösung gepuffert.

Eine weitere Verbesserung der Lagerungsfähigkeit der Erythrozyten lässt sich dadurch erreichen, dass man den vorstehend genannten Mischungen ein Kolloid, beispielsweise modifizierte Gelatine, Dextran, Hydroxyethylstärke oder Serumalbumin zusetzt (vgl. Ullmann, 4. Aufl., B. 8 (1974), S. 634-640).

NaCl kann zur Erzielung der Isotonie eingesetzt werden.

Typische Lösungen setzen sich beispielsweise wie folgt zusammen:
Pro 1 000 ml gelöst in aqua ad injectabilia

A. 0 bis 5 g Natriumchlorid
0 bis 5 g Di-Natriumhydrogenphosphat
0 bis 5 g Natriumdihydrogenphosphat
0,5 bis 20 g Glucose oder Fructose
mehr als 110 mmol Sorbit, Mannit oder Xylit
0 bis 1 g Adenin
0 bis 1,5 g Guanosin
0 bis 10% Kolloide

Vorzugsweise:

B. 0 bis 2,5 g Natriumchlorid
0,7 bis 2,5 g Di-Natriumhydrogenphosphat
0,7 bis 2,5 g Natriumdihydrogenphosphat
5 bis 15 g Glucose oder Fructose, vorzugsweise Glucose
220 bis 330 mmol = ca. 40 bis 60 g Sorbit, Mannit oder Xylit, vorzugsweise Sorbit
ggf. mit folgenden zusätlichen Substanzen:

C. 0,1 bis 0,5 g Adenin
0,1 bis 0,5 g Guanosin

Eine besonders bevorzugte Lösung enthält:

D. 1,23 g Natriumchlorid
1,11 g Natriumdihydrogenphosphat $\bullet$ H$_2$O
2,88 g Di-Natriumhydrogenphosphat $\bullet$ 12H$_2$O
9,4 g Glucose-Monohydrat
50 g = 275 mmol Sorbit
und ggf. zusätzlich:

E. 0,246 g Adeninhydrochlorid
0,408 g Guanosin

Bei der Anwendung dieser Lösungen wird üblicherweise so verfahren, dass zunächst ein mit einer ACD-, CPD- Citrat- oder Heparin-Lösung (USP XX (1980), S. 49 - 50) stabilisiertes Vollblut hergestellt wird. Die Vollblutkonserve wird zentrifugiert und anschliessend das Plasma abgetrennt. Anschliessend wird vorzugsweise im geschlossenen, aus mehreren Beuteln bestehenden System zum Erythrozytenkonzentrat Suspensionslösung zum Waschen oder zum Lagern zugegeben. Zum Beispiel können ca. 200 - 300 ml Erythrozytenkonzentrat mit HK von 60 - 85, ca. 50 bis 250 ml Suspensionslösung in einer oder mehreren Portionen zugegeben werden. Die Mischung kann dann wie bisher eine Blutkonserve im Kühlschrank bei ca. 6°C gelagert oder auch direkt transfundiert werden.

Die erfindungsgemässen Lösungen können auch zur Waschung und Konservierung von Erythrozyten verwendet werden.

Gleichzeitig sind diese Mischungen zur Transfusion geeignet. Bei der Anwendung dieser Lösungen als Waschlösung werden z.B. zu 250 ml Erythrozytenkonzentrat 50 bis 250 ml dieser Lösungen, vorzugsweise 70 ml, hinzugegeben. Die Mischungen werden zentrifugiert und der Überstand abgetrennt. Dieser Vorgang kann 2 bis 3 mal wiederholt werden, so dass beim Waschprozess bis zu 210 ml dieser Lösungen notwendig sind. Anschliessend kann das Erythrozytenkonzentrat zusammen mit der Suspensionslösung gelagert oder aber mit oder ohne Suspensionslösung sofort transfundiert werden.

Die oben beschriebenen Lösungen werden im Dreifach- oder Vierfach-Beutelsystem abgefüllt bestehend aus:

1. Beutel 450 ml mit 65 ml CPD-Stabilisator
2. Beutel 450 ml mit 100 ml der beschriebenen Lösung
3. Beutel 300 ml leer
ggf. 4. Beutel 300 ml leer
Die Beutel werden autoklaviert.

Beispiel 1

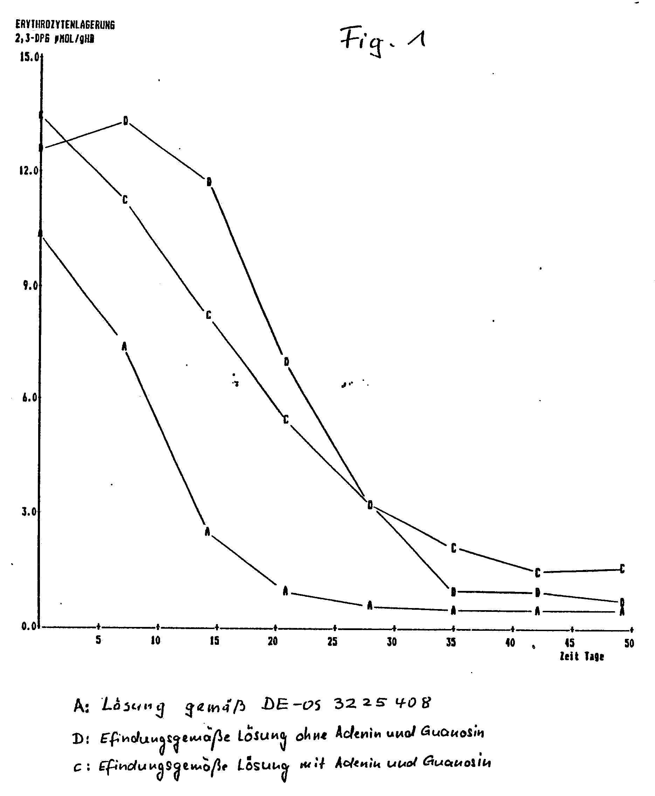

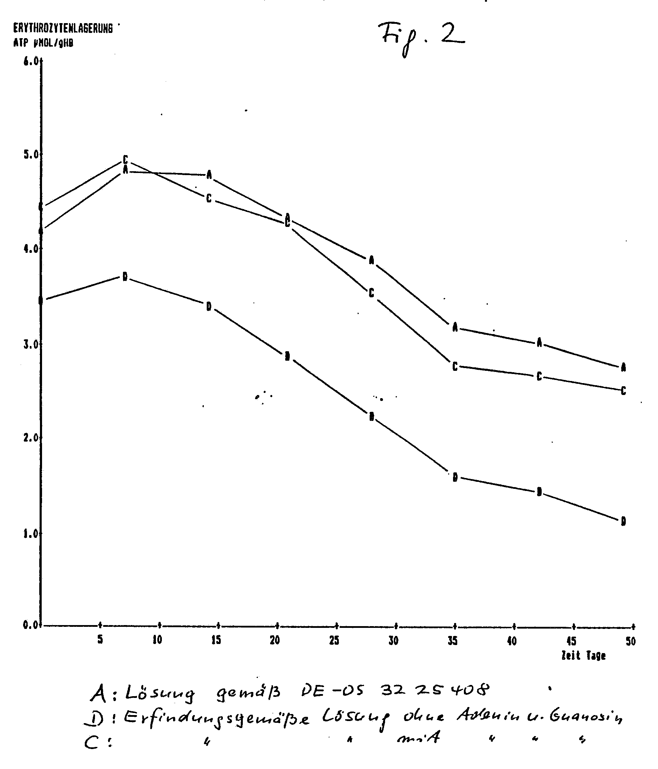

In einem oben beschriebenen Dreifach-Beutel wurden 450 ml Blut unter sorgfältigem Durchmischen gespendet. Die Konserve wure anschliessend bei 4°C und 3 500 g für 10 Minuten zentrifugiert. Nach Abtrennen des Plasmas und des buffy-coat in den 300 ml Leerbeutel wurden 100 ml der vorstehend als D. bezeichneten Suspensionslösung ohne Adenin und Guanosin zu den Erythrozyten gegeben. Die Konserve wurde bei 4°C gelagert. Es wurde festgestellt, dass bei dieser Lösung der 2,3-DPG-Gehalt bis zum 28. Tag der Lagerung höher war als bei einer aus der DE-OS 32 25 408 bekannten Lösung folgender Zusammensetzung: Pro 1 000 ml gelöst in aqua ad injectabilia: 4,21 g NaCl; 1,11 g NaH$_2$PO$_4$ • H$_2$0; 2,88 g Na$_2$HP0$_4$ • 12H$_2$0; 9,4 g Glucose-Monohydrat; 10 g = 55 mmol Sorbit; 0,246 g Adeninhydrochlorid; 0,408 g Guanosin, die zum Vergleich herangezogen wurde (siehe Fig. 1). Der ATP-Wert war etwas niedriger (siehe Fig. 2).

Biespiel 2

Die Versuchsdurchführung war analog dem Beispiel 1, jedoch kam die Suspensionslösung E. zum Einsatz, d.h. mit zusätzlichem Adenin und Guanosin.

Auch bei diesen Konserven trat eine Verbesserung des 2,3-DPG-Gehaltes auf gegenüber der bekannten Lösung (siehe Fig. 1). Der ATP-Wert war nahezu identisch zu dieser Lösung (siehe Fig. 2).

Ansprüche

1. Wässrige Lösung zum Suspendieren und Lagern von Zellen, insbesondere Erythrozyten, enthaltend mindestens einen Nährstoff und mindestens eine nicht in die Zellen eindringende, in wässriger Lösung lösliche Substanz, dadurch gekennzeichnet, dass diese nicht in die Zellen eindringende, in wässriger Lösung lösliche Substanz in einer Konzentration von mehr als 110 mmol/l vorliegt.

2. Lösung nach Anspruch 1, dadurch gekennzeichnet, dass die nicht in die Zellen eindringende Substanz ein Zuckeralkohol ist.

3. Lösung nach Anspruch 2, dadurch gekennzeichnet, dass der Zuckeralkohol Sorbit, Mannit oder Xylit ist.

4. Lösung nach einem der vorstehenden Ansprüche, dadurch gekennzeichnet, dass der Nährstoff Glucose oder Fructose ist.

5. Lösung nach einem der vorstehenden Ansprüche, dadurch gekennzeichnet, daß sie außerdem Adenin und/oder Guanosin enthält.

6. Lösung nach einem der vorstehenden Ansprüche, dadurch gekennzeichnet, dass sie ausserdem ein Kolloid enthält.

7. Lösung nach einem vorstehenden Ansprüche, dadurch gekennzeichnet, dass sie pro 1 aqua ad injectabilia
0 bis 5 g Natriumchlorid
0 bis 5 g Di-Natriumhydrogenphosphat
0 bis 5 g Natriumdihydrogenphosphat
0,5 bis 20 g Glucose oder Fructose
mehr als 110 mmol Sorbit, Mannit oder Xylit
0 bis 1 g Adenin
0 bis 1,5 g Guanosin und
0 bis 10% Kolloide
enthält.

8. Lösung nach Anspruch 7, dadurch gekennzeichnet, dass sie pro 1 aqua ad injectabilia
1,23 g Natriumchlorid
1,11 g Natriumdihydrogenphosphat • H$_2$0
2,88 g Di-Natriumhydrogenphosphat • 12H$_2$0
9,4 g Glucose-Monohydrat
50 g = 275 mmol Sorbit
enthält.

9. Lösung nach Anspruch 8, dadurch gekennzeichnet, dass sie ausserdem
0,246 g Adeninhydrochlorid und
0,408 g Guanosin
enthält.

10. Verwendung einer Erythrozyten-Suspensionslösung nach einem der vorstehenden Ansprüche zur Verbesserung der Sauerstoffabgabe der Erythrozyten.

Fig. 1

A: Lösung gemäß DE-OS 32 25 408

D: Efindungsgemäße Lösung ohne Adenin und Guanosin

c: Efindungsgemöße Lösung mit Adenin und Guanosin

Fig. 2

A: Lösung gemäß DE-OS 32 25 408
D: Erfindungsgemäße Lösung ohne Adenin u. Guanosin
C:          "                "        mit A     "     "     "

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS, Band 99, Nr 3, 18. Juli 1983, Seite 440, Ref.Nr. 20367x, Columbus, Ohio, USA; & SU-A-1 009 469 (M.M. PETROV et al.) 07-04-1983 * Zusammenfassung * | 1-4,7, 10 | A 01 N 1/02 A 61 K 35/18 |
| Y | Idem --- | 5,6,8,9 | |
| Y,D | DE-A-3 225 408 (W.H. WALKER) * Ansprüche; Seite 4, Zeile 30 - Seite 6, Zeile 14 * --- | 5,6,8,9 | |
| X | CHEMICAL ABSTRACTS, Band 103, Nr. 20, 18. November 1985, Seite 358, Ref.Nr. 165990f, Columbus, Ohio, US; N.R. GUSEVA et al.: "Effect of the composition of resuspension media on the preservation of thawed erythrocytes at +4 C." & KRIOBIOLOGIYA 1985, (2), 32-7 * Zusammenfassung * --- | 1-4,7, 10 | |
| X | CHEMICAL ABSTRACTS, Band 88, Nr. 19, 8. Mai 1978, Seite 303, Ref.Nr. 133872z, Colombus, Ohio, US; Y. DOI et al.: "Applcation of various frozen stored animal red blood cells for the virological and serological tests" & UIRUSU 1977, 27(2), 79-89 * Zusammenfassung * --- | 1-4 | RECHERCHIERTE SACHGEBIETE (Int. Cl.4)<br><br>A 01 N A 61 K |
| Y | EP-A-0 099 315 (CENTRE REGIONAL DE TRANSFUSION) * Ansprüche * --- -/- | 1-5,7, 10 | |

Der vorliegende Recherchenbericht wurde für alle Patentansprüche erstellt

| Recherchenort | Abschlußdatum der Recherche | Prüfer |
|---|---|---|
| DEN HAAG | 04-11-1988 | FLETCHER A.S. |

Europäisches Patentamt

# EUROPÄISCHER RECHERCHENBERICHT

Nummer der Anmeldung

EP 88 11 0246

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DD-A- 152 719 (BEZIRKSINST. FÜR BLUTSPEND. UND TRANSFUSIONWESEN) * Ansprüche * --- | 1-5,7, 10 | |
| D,A | US-A-4 267 269 (G.A. GRODE et al.) * Spalte 1, Zeilen 45-54; Ansprüche * ----- | 1-10 | |

RECHERCHIERTE SACHGEBIETE (Int. Cl.4)

Der vorliegende Recherchenbericht wurde für alle Patentansprüche erstellt

| Recherchenort | Abschlußdatum der Recherche | Prüfer |
|---|---|---|
| DEN HAAG | 04-11-1988 | FLETCHER A.S. |